# EUROPEAN PATENT SPECIFICATION

(11) **EP 3 203 867 B1**
(45) Date of publication and mention of the grant of the patent: **24.04.2019**
(21) Application number: 14787306.1
(22) Date of filing: 07.10.2014
(51) Int. Cl.: A42B 1/06

(54) **HEAD PIECE ADAPTED FOR USE WITH AND WITHOUT EARMUFFS.**
ZUR VERWENDUNG MIT UND OHNE OHRSCHÜTZER ANGEPASSTES KOPFSTÜCK
CASQUE ADAPTÉ À UNE UTILISATION AVEC ET SANS PROTÈGE-OREILLES

(43) Date of publication of application: 16.08.2017
(73) Proprietor: LILJEBLAD, Jonas, 13235 Saltsjö-Boo (SE)
(72) Inventor: LILJEBLAD, Jonas, 13235 Saltsjö-Boo (SE)
(74) Representative: Ehrner & Delmar Patentbyrå AB
(86) International application number: PCT/SE2014/000126
(87) International publication number: WO 2016/056950

(56) References cited:
- JP-A- 2008 007 864
- US-A- 1 879 104
- US-A1- 2006 288 468
- US-A1- 2009 257 615
- US-B1- 8 769 723

## Description

The invention relates to a head piece adapted to be worn with and without earmuffs. Specifically the invention relates to a head piece that may be used in two different modes, wherein it is adapted to be worn without earmuffs in a first mode and with earmuffs in a second mode.

### Background

Many outdoor activities, such as lawn mowing, hunting, construction work etc. produce noise and loud sounds and therefore require the use of sound protectors. The failure to use sound protectors, such as earmuffs, may seriously harm the hearing ability. It is both a health and security risk to be exposed to loud noise, even if only for a very short period of time. The exposure to loud noise reduces a person's ability to concentrate, whereby the risk of injuries due to lack of concentration is substantially increased. Exposure to sounds in excess of 85 db for extended periods of time can result in long term hearing loss. Most countries apply laws and/or standards to make sure that workers' that are regularly or at times exposed to sounds in excess of 85 db need to wear effective sound protectors. Often it is the responsibility of the employer to make sure that workplace-caused harms including long term hearing loss are avoided.

Many of such sound producing outdoor activities are performed in all climates and regardless of the outside temperature. Therefore, in cold climates, there is a need to use a head piece to keep the head and ears warm at the same time as the earmuffs are used. Earmuffs are designed to fit closely around the ears of the person wearing them such that the rims of the earmuffs lie firmly against the head of the person wearing them.

Incorrect use of earmuffs may jeopardize the safety and health of the wearer substantially. Various solutions involving hats or head pieces that are especially adapted to be used with earmuffs have therefore been attempted but have not met with success in the marketplace.

IN US 2006/0288468 A1 a head piece is disclosed which is adapted to be used with earmuffs. The head piece may be used inside a helmet or without a helmet, but with earmuffs. The head piece is not adapted to be used without earmuffs, since such use would leave the ears uncovered.

In SE 530627 C2 a different type of head piece is disclosed, which is also adapted to be used with earmuffs, but which does not include an opening in the head piece that enables the earmuff direct contact with the head for the wearer. Instead the head piece includes a thin membrane that allows the earmuffs to come relatively close to the skin of the person wearing them. This head piece is a compromise that depending on the texture of the membrane does not provide sufficient sound protection for the ears, and/or does not keep the ears warm when the earmuff is not worn.

Another approach is presented in US 2009/0199326 A1. In this a hard hat is provided with retractable ear plugs. Ear plugs do however have some disadvantages in comparison to earmuffs. Firstly, they may not be as effective to attenuate noise. Further, they are not as hygienic as they are entered into the ear of the user. They spread diseases and/or bacteria between different user and they will easily be exposed to filth such as dust, especially when not in use. The earplugs should therefore be personal, i.e. used by one person only, and even so they need to be replaced at regular intervals for hygienic reasons. Further, the earplug solution does not help to keep the ear portion of the user warm, neither when in use nor when not in use.

None of these head pieces are hence well adapted to be used in a cold climate both with and without earmuffs.

In some activities, such as hunting and forest work the earmuffs are only needed for a short period of times and at specific moments. In such activities it would be desirable to put on and take off the earmuffs several times during a regular work shift. Systems which fail to provide for this flexibility increase the risk that ear protection will not be worn. Therefore it would be advantageous to have a head piece that offers rapid adjustment between a mode in which no earmuffs are worn and a mode in which earmuffs are worn.

In US 2009/0257615 a headwear is described, which is adapted to be used with in-ear headphones, specifically in-ear headphones that are interconnected by an arc-shaped loop arranged over the head. The headwear is provided with small openings that are located above the the ears of the wearer that allows entrance of the in-ear headphones and the lower part of the loop. This construction is however not adaptable to the use of earmuffs, because the size of earmuffs would imply that the openings should be very big and that there would be a great space arranged around the ear portions of the headwear to allow housing of earmuffs inside the headwear. Furthermore, such a solution with a large opening in the headwear would lead to deficiencies in the protection against cold and precipitation when wearing earmuffs, because it would be problematic to reseal the opening above the earmuffs.

Hence, there is a need for a head piece that is well adapted for use both with and without earmuffs, i.e. a head piece which keeps the wearer's ears warm when the earmuffs are not in use, and which allows the optimum sound reduction of the earmuffs when they are used. Preferably, the head piece should be relatively easy to adjust between the two modes of use.

### Summary of the invention

An object of the invention is to provide a head piece that is well adapted for use both with and without earmuffs.

This object is achieved by the invention according to claim 1, which relates to a head piece adapted to be worn with and without earmuffs, which head piece includes ear portions adapted to cover the ears of a person wearing the head piece. The ear portions include at least one cover piece that may be placed in an open mode in which it allows access of an earmuff through an opening in the ear portions of the head piece, wherein the cover piece when in a closed mode covers the ear portion so as to keep the ears of a person wearing the head piece covered.

The two modes of the head piece allows it to be easily adapted between an ordinary head piece that keeps the head and ears of a wearer warm, and a head piece that is adapted to be used with earmuffs to provide optimum protection from loud and harmful noise.

In a specific embodiment of the invention the cover piece has an outer lining arranged to face the outside of the head piece and an inner lining arranged to face the inside of the head piece, and wherein the inner lining is prevented from exposure to the outside both in the closed and the opened mode.

With this specific embodiment the inner lining is never exposed to the surrounding environment, such that snow, rain, debris and the like will be kept off the inner lining. Hence, the head piece may be adapted between the two modes even in harsh conditions and without jeopardising its well function in either mode.

Specifically the inner lining of the cover piece may be arranged so as to face the head, or the skin, of the wearer both in the closed and the open mode.

In another specific embodiment of the invention the opening is, when the head piece is worn on a person's head, arranged to be located around the ears of said person to allow access of a complete rim of an earmuff through the opening.

With this specific embodiment it is made sure that the complete rim is allowed through the opening in order to guarantee optimal contact between the earmuff and the head of the wearer. Such specific fitting may, in order to provide a close and warm keeping fit, demand that the opening is adapted to one specific type of earmuff, since the size and shape of an earmuff may vary between different manufacturers.

Other features and advantages of the invention will be apparent from the figures and from the detailed description of the shown embodiment.

The head piece may be fabricated in any suitable materials including but not limited to such materials as acrylic knit, polyester fleece, wool, silk, olefin, nylon, rayon, neoprene, dryfit, goretex, cotton knit, cotton canvas, etc.

### Short description of the drawings

In the following detailed description reference is made to the accompanying drawings, of which:
- **Fig. 1**: shows a first embodiment of a head piece according to the invention in a closed mode;
- **Fig. 2**: shows the embodiment of figure 1 in an open mode;
- **Fig. 3**: shows the embodiment of figure 1 in an open mode and with earmuffs;
- **Fig. 4**: shows a second embodiment of a head piece according to the invention in a closed mode;
- **Fig. 5**: shows the embodiment of figure 4 in an open mode and with earmuffs;
- **Fig. 6**: shows a third embodiment of a head piece according to the invention in a closed mode;
- **Fig. 7**: shows the embodiment of figure 6 in an open mode and with earmuffs;
- **Fig. 8**: shows a fourth embodiment of a head piece according to the invention in an open mode;
- **Fig. 9**: shows the embodiment of figure 8 in an open mode, with earmuffs and with the cover pieces as face protection;
- **Fig. 10**: indicates the geometry of the embodiment of figure 8; and
- **Fig. 11**: shows the embodiment of figure 8 in a closed mode.

### Detailed description of the shown embodiment of the invention

In the following description similar features have been given the same reference numerals even though they are not identical.

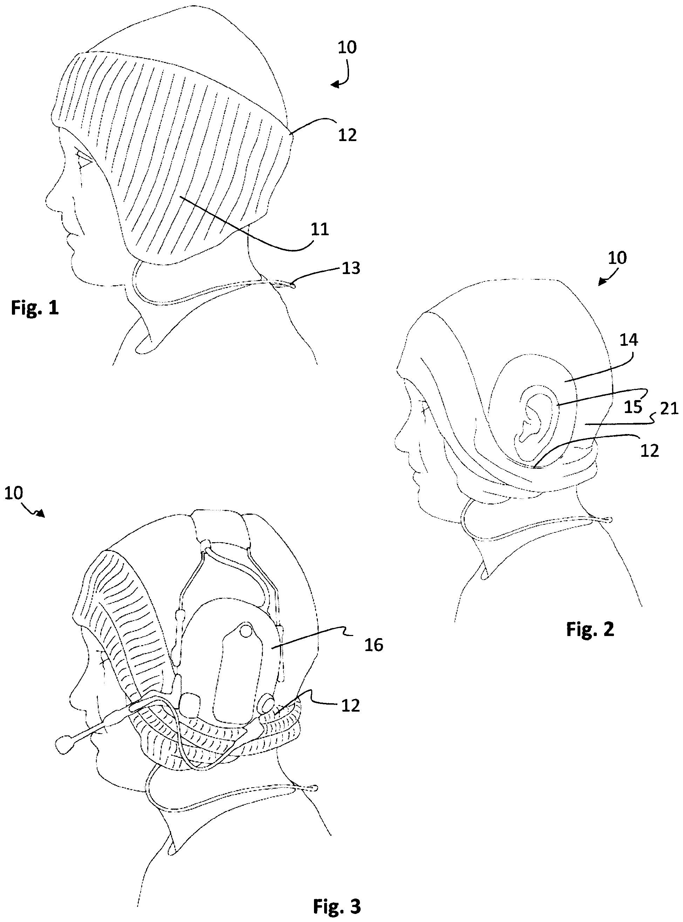

In figures 1-3 a first embodiment of the head piece 10 according to the invention is shown. The innovative head piece 10 is adapted to be worn both with and without earmuffs 16. The head piece 10 includes ear portions 11 that are adapted to cover the ears of a person wearing the head piece 10. The ear portions 11 include at least one flap or cover piece 12 that may be placed in an open mode in which it allows access of an earmuff 16 through the ear portions 11 of the head piece, further providing that the cover piece 12 when in a closed mode covers the ear portion 11 so as to keep the ears of a person wearing the head piece 10 covered. A strap 13 that connects the ear portions 11 is arranged in the shown first embodiment. The strap 13 may be bound or fastened below the chin of the wearer so as to tighten the ear portions against the cheek of the wearer.

In the first embodiment of the head piece 10, the ear portion 11 of the head piece 10 comprises two layers, an inner layer 21, and an outer layer that forms a cover piece 12. The inner layer 21 of the ear portion 11 has an opening 14 adapted to the size of an earmuff 16, and the cover piece 12 forms an outer layer that covers said opening 14 in the closed mode but that may be pulled down in the open mode so as to allow access of an earmuff 16 through said opening 14 in the inner layer 21.

The outer layer may comprise a supplementary liner (not shown) positioned so as to provide a second layer adapted to cover the opening 14 of the inner layer 21 in the closed mode. This supplementary liner preferably has a similar shape as the opening 14 such that it will fit closely inside the opening 14 and such that the whole ear portion will be covered by a double layer in the closed mode.

Preferably, a seam (not shown) that connects the inner layer 21 to the cover piece is provided in front of the opening 14. This seam makes sure that the cover piece will not fold down too low so as to interfere with the sight of the person wearing the head piece. The exact position and shape of the seam may vary from design to design to optimize this functional aspect.

In fig. 2 the cover piece has been pulled down such that the opening 14 is exposed. As is apparent from the figure the cover piece 12 is mainly pulled down below the opening 14 and behind the opening, but not as much in front of the opening. This is due to the seam joining the inner layer and the cover piece 12.

From fig. 3 it is visible that the cover piece 12 may be arranged so as to cover a lower part of the earmuff 16. The head piece according to the first embodiment is well adapted to be worn both with and without earmuffs.

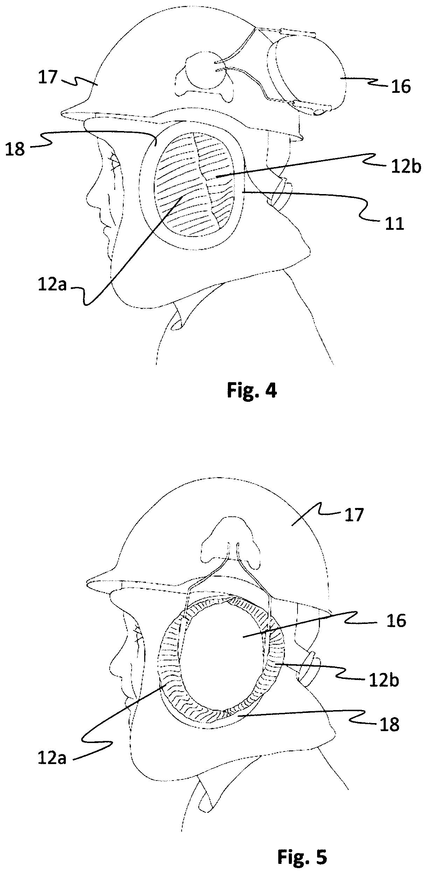

A second embodiment of the invention is shown in figures 4-5. In this embodiment each ear portion 11 includes a front cover portion 12a and a back cover portion 12b which may be attached to each other in said closed mode in order to cover the ears of the person wearing the head piece 10, as is shown in figure 4. The cover portions 12a,b of this second embodiment may preferably be arranged in an elastic material, such that they may be manually adjusted. In the shown embodiment a rim portion 18 is arranged to separate the cover portions 12a,12b from the rest of the head piece 10. This rim portion 18 may assist in adding structure and/or stiffness to the ear portion 11 of the head piece. It may however be dispensed with without losing the function of the cover portions 12a,12b. Also, the rim portion 18 may be made invisible, i.e. with the seam provided on the inside of the head piece 10.

The cover portions 12a,12b may be separated from each other so as to allow access of an earmuff 16 between said cover portions 12a,12b, as is shown in figure 5. The cover portions 12a,12b may preferably be arranged and be of a stretch material so as to fit tightly around the earmuffs 16, such that wind, rain, snow and the like will not reach through the gap between the cover portion 12a,b and an earmuff 16.

To simplify putting on the earmuffs, the front cover portions 12a overlaps the back cover portions 12b. This allows the earmuff to catch and separate the front cover portions 12a from the back cover portions 12b when the earmuffs are rotated down from the position they have when the earmuffs are not in use.

The cover portions 12a,12b may include magnets for attachment to each other so as to provide a complete cover for the ears. Preferably, each cover portion 12a,12b includes two magnets. It may however also be sufficient with one magnet, and it may be possible to use three or more magnets. The optimal number of magnets is dependent on the stiffness of the material of the cover pieces. If the material is not particularly stiff and the number of magnets is great there is a risk that the magnets will bond to incorrect magnets, i.e. not the intended magnet.

The cover portions 12a,12b may also include Velcro for attachment of the cover portions 12a,12b to each other. In contrast to the magnets there is no conflict in using Velcro pieces located close to each other, as long as there is sufficient material in between the Velcro pieces to allow the cover portions 12a,12b to stretch around an earmuff.

In yet another embodiment buttons may be used to attach the cover portions 12a,12b to each other. In preferable embodiments one, two or three buttons are used. The buttons may e.g. be snap buttons or sew-on buttons of a shirt button type.

The foregoing discussion of fastening methods is meant to be illustrative but not exhaustive, and any reasonable method of attaching the cover portions so that they are secure over the openings in the head piece when the earmuffs are not in use is considered to be within the scope of the present invention.

The head piece according to the second embodiment is well adapted to be worn both with and without earmuffs. Furthermore the ear cover portions of the second embodiment as shown are designed to make the head piece compatible with a hard hat or a helmet. The head piece may e.g. be of a balaclava type.

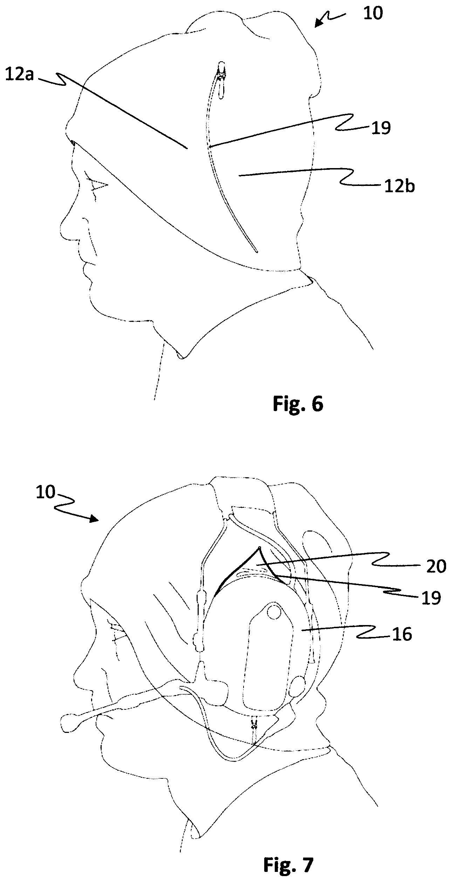

A third embodiment of the invention is shown in figures 6-7. In this embodiment each ear portion 11 includes a front cover portion 12a and a back cover portion 12b that are separated from each other by means of a zipper 19. The zipper 19 may be opened to allow access of an earmuff through a zipper opening, and closed to cover the ear portions 11 so as to keep the ears of a person wearing the head piece 10 covered. As is apparent in the figures the zipper 19 is arranged in an arc shape. The arc shape assist in creating a big enough opening so as to allow the access of an earmuff 16. Further, the zipper 19 preferably has a forward arc shape, meaning that the major part of the cover portion 12a,12b will be pulled backwards, behind the wearer's ears, in the open mode. In figure 6 this is apparent since the front cover portion 12a is smaller than the back cover portion 12b. With such a forward arc shape there will be less material in front of the opening, which in turn reduces the risk of any material of the head piece will come into or near the eye of the wearer.

An ordinary straight zipper may also be used as long as the material of the head piece is elastic enough so it enables the wearer to adjust the front cover portions by pulling the top of the hat back, this to prevent the front cover portions from interfering with the eyesight of the wearer. However, as long as an elastic material is chosen it may be easier to produce a head piece with a good fit with a straight zipper that with an arc shaped zipper.

Another way of creating a big enough zipper opening is to have one end, preferably the lower end of the zipper open, much like the upper end of a fly of pair of pants. In such an embodiment a straight zipper would be preferred. It would however be preferable to have a second layer below the outer layer such that the portion around the earmuffs will be covered even when the zipper is open. Also, an ordinary straight zipper may be used as long as the material of the head piece is elastic enough so as to allow the creation of a sufficient opening.

In the embodiment shown in in figures 6-7 the zipper opening comprises a tongue part 20 that covers an end portion of the zipper opening 14. In the shown embodiment the tongue part 20 covers the upper end portion. In a way, the tongue part 20 constitutes an inner layer at a portion of the opening. The function of the tongue part 20 is to allow the use of a long enough zipper while at same time making sure that a gap will not be created around the earmuff in the open mode.

The head piece according to the third embodiment is well adapted to be worn both with and without earmuffs.

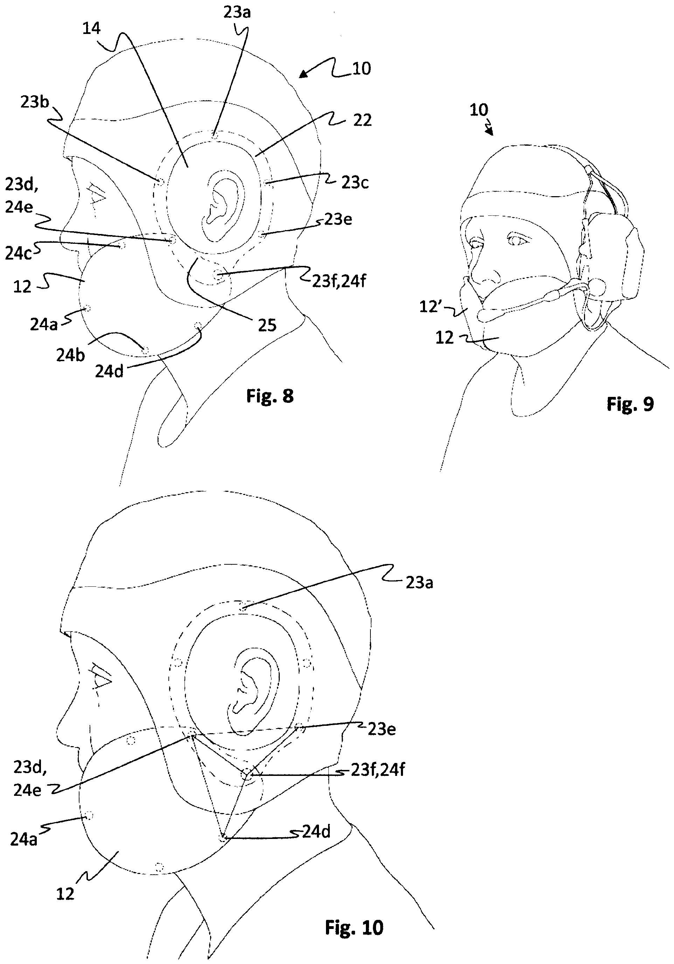

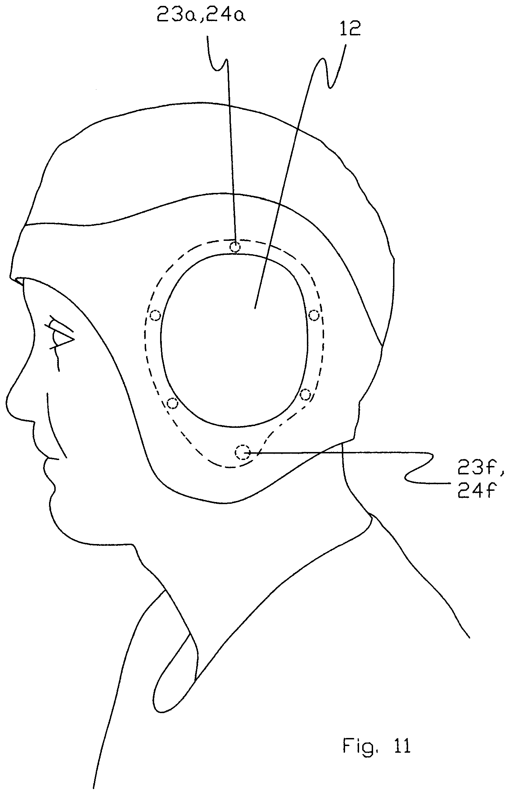

A fourth embodiment is shown in figures 8-11. In this embodiment the cover piece 12 is rotatably arranged and may be positioned in a closed position (fig. 11) where it covers an opening 14 in the ear portion 11 and an open position where it does not cover the opening 14, but allows access of an earmuff 16 through said opening 14.

For this, each cover piece 12 comprises attachment pieces 24a-f that allow the cover piece 12 to be fixed to the ear portion 11 of the head piece 10 in corresponding attachment pieces 23a-f. In figures 8-10 the cover pieces 12 are shown in an open mode with the cover pieces 12 tilted to a forward position where they may meet below the mouth of the user. In figure 9 it is illustrated how a left cover piece 12 may be attached to a right cover piece 12'. In a closed mode an attachment piece 23a of the head piece will be attached to an attachment piece 24a of a corresponding reference letter on the cover piece.

Depending on the choice of attachment method, there may be a need to complement attachment points 24a with additional fasteners suitable for the purpose. This is to ensure that the cover pieces do not separate from each other when attached in the open position. Appropriate complementary attachment method could be, but are not limited to, a loop and button or a hook and eye.

In the shown embodiment a rim portion 22 including the attachment pieces 23a-f is arranged as a border around the opening 14.

The cover piece 12 is rotatable around a lower attachment point corresponding to an attachment piece 23f,24f. Preferably, the cover piece 11 is undetachable from or permanently attached to the head piece in the lower attachment piece 23f,24f. This is useful since it makes sure that the cover pieces will not be lost. However, it may also be useful be able to completely release the cover pieces from the ear portions. In such a case all attachment pieces including the lower attachment piece 23f,24f releasable. Further, if the attachment pieces are compatible with each other, e.g. of a male and female type, the cover pieces may be attached to each other and kept e.g. in the wearer's pocket, or in a pocket specially arranged on the headpiece.

As apparent from the figures 8-10 both cover pieces 12 comprise an upper attachment piece 23a by mean of which the cover pieces 11 may be attached to each other in the open mode. In the figures 8-10 the cover pieces 12 are attached to each other below or in front of the mouth of the user. They may however just as well be attached to each other behind the ears of the wearer, i.e. in the neck portion, to keep the neck of the wearer warm or below the chin of the wearer as a scarf. Further, the cover pieces 12,12' may be arranged so as to hang freely along the side of the wearer's head attached only in the lower attachment point 23f,24f. The fourth embodiment may of course be combined with a balaclava type head piece, in which the cover pieces could be arranged downwards in the open mode. Possible an attachment piece could be included to attach the cover pieces downwards in the closed mode.

In order for the cover piece 12 not to cover the opening 14 when in the open mode it comprises a recessed portion 25, which in the shown embodiment consists of an inwardly shaped arc. See fig. 8. A corresponding recessed portion is not needed on the opposite side since the cover piece 12 takes a lower position out of the opening when tilted backwards towards the neck portion.

As illustrated in figure 10 the attachment pieces are so arranged with respect to each other that the cover piece 12 may be attached in both the closed and the open mode. This is possible since the distances between lowest attachment point 23f,24f, around which the cover piece may be rotated, and the two closest left attachment pieces 23d,24d and the two closest right attachment pieces 23f,24f, respectively, are the same. Thereby, the lower back attachment piece 24e of the cover piece 12 may be attached to the lower front attachment piece 23d of the head piece, such that the cover piece 12 will be rotated forward so as to cover the chin of the wearer. In this position the upper attachment pieces 24a of the cover pieces 12 and 12', respectively, may be attached to each other. Of course, for this to work the buttons need to be compatible with each other.

In a preferred embodiment one cover piece has only male attachment pieces, wherein the other has only female attachment pieces, and wherein the corresponding attachment pieces of the head piece are arranged to mate with the corresponding cover piece. In this manner the right cover piece may only be attached to the right side of the head piece, the left cover piece may only be attached to the left side of the head piece, while at the same time allowing the cover pieces to be attached to each other. As previously discussed attachment pieces and methods may include but are not limited to magnets, snap buttons, shirt buttons, Velcro or the like.

Also, the lower front attachment piece 24d of the cover piece 12 may be attached to the lower back attachment piece 23e of the head piece, such that the cover piece 12 will be rotated backward so as to cover the neck of the wearer. The angles between these three attachment points 23-24e,d,f will decide at which position the cover piece 12 will end up in the different positions. Numerous angles to suit the circumstance and contemplated user preference are considered to be within the scope of the present invention.

In figure 11 the cover piece 12 is shown in the closed position in which position each attachment piece 24a-f of the cover piece 12 is positioned so as to be attached to a corresponding attachment piece 23a-f of the ear portion of the head piece.

The head piece according to the forth embodiment is well adapted to be worn both with and without earmuffs. Furthermore the ear cover portions of the forth embodiment as shown are designed to make the head piece compatible with a hard hat or a helmet. The head piece may e.g. be of a balaclava type.

The invention has been described above with reference to four specific embodiments. The invention is however not limited to these embodiments. The invention comprises further embodiments which are considered to be within the scope of protection as defined by the following claims.

Specifically, above the different embodiments of the head piece according to the invention have been described to be adapted to earmuffs. The inventive head piece is however also useful to be worn with headphones of the around ear type. Normally, earmuffs are larger and have an oval form whilst headphones are often circular and somewhat smaller. Around-ear headphones are however, as are earmuffs, adapted to be in contact with the ear or the area around the ear in order to keep out sounds from the surroundings. Therefore the head piece according to the invention is just as advantages when it comes to wearing head phones as when wearing earmuffs in cold environments.

## Claims

1. A head piece (10) adapted to be worn both with and without earmuffs (16), which head piece (10) includes two opposed ear portions, which when the head piece (10) is worn on a person's head (11) is adapted to cover the ears of said person, **characterised in that** each ear portion (11) includes at least one cover piece (12,12',12a,12b) that may be adjusted between an open mode in which it allows access of an earmuff (16) through an opening (14) in the ear portions (11) of the head piece such that rims of the earmuffs may lie firmly against the head of the person wearing them, and a closed mode in which the cover piece (12,12',12a,12b) covers the ear portion (11) so as to keep the ears of said person covered.

2. The head piece (10) according to claim 1, wherein the cover piece (12,12',12a,12b) has an outer lining arranged to face the outside of the head piece and an inner lining arranged to face the inside of the head piece, and wherein the inner lining is prevented from exposure to the outside both in the closed and the open mode.

3. The head piece (10) according to claim 3, wherein the inner lining of cover piece (12,12',12a,12b) is adapted to face the head of the wearer both in the closed and the open mode.

4. The head piece (10) according to anyone of the preceding claims, wherein at least the ear portions (11) of the head piece (10) comprise two layers, wherein an inner layer (21) of the two layers has an opening (14) adapted to the size of an earmuff (16), and wherein the cover piece (12) forms an outer layer that covers said opening (14) in the closed mode but that may be pulled down in the open mode so as to allow access of an earmuff (16) through said opening (14) in the inner layer (21).

5. The head piece (10) according to claim 4, wherein the outer layer comprises a supplementary liner positioned so as to provide a second layer adapted to cover the opening (14) of the inner layer (21) in the closed mode.

6. The head piece (10) according to anyone of claims 1, 2 or 3, wherein each ear portion (11) includes two cover portions (12a,12b) which may be joined to each other in said closed mode in order to cover the ears (15) of a person wearing the head piece (10) and which may be separated from each other so as to allow access of an earmuff (16) between said two cover portions (12a,12b).

7. The head piece (10) according to claim 6, wherein the cover portions (12a,12b) include attachment pieces for attachment of the cover portions (12a,12b) to each other.

8. The head piece (10) according to claim 7, wherein the attachment pieces include Velcro, snap buttons, shirt buttons and/or magnets.

9. The head piece (10) according to claim 6, wherein the ear portions (11) include a zipper (19) for attachment of the cover portions (12a,12b) to each other, which zipper (19) may be opened to allow access of an earmuff through a zipper opening, and closed to cover the ear portions (11) so as to keep the ears of a person wearing the head piece covered.

10. The head piece (10) according to claim 9, wherein the zipper opening (14) comprises a tongue part (20) that covers an end portion of the zipper opening (14).

11. The head piece (10) according to either of claims 9 or 10, wherein the zipper (19) is arc shaped, such that a front cover portion (12a) of the two cover portions (12a,12b) is smaller than a back cover portion (12b).

12. The head piece (10) according to anyone of claims 1, 2 or 3, wherein the cover piece (12,12') is rotatably arranged and may be positioned in a closed position where it covers an opening (14) of the ear portion (11), and an open position where it does not cover the opening (14) but allows access of an earmuff (16) through said opening (14).

13. A head piece (10) according to claim 12, wherein each cover piece (12,12') comprises attachment pieces (24a-f) that allow it to be fixed to corresponding attachment pieces (23a-f) arranged at the ear portion (11) of the head piece (10).

14. A head piece (10) according to claim 13, wherein each cover piece (12,12') may be fixed both in the closed and in the open position to the attachment pieces (23a-f) of the ear portion (11) of the head piece (10).

15. A head piece (10) according to any of the claims 12-14, wherein each cover piece (12,12') is rotatable around a lower attachment point (23f,24f) located below the opening (14) of the ear portion (11).

## Patentansprüche

1. Kopfbedeckung (10), die geeignet ist, sowohl mit als auch ohne Gehörschutzkapseln (16) getragen zu werden, wobei die Kopfbedeckung (10) zwei sich gegenüberliegende Ohrabschnitte umfasst, die, wenn die Kopfbedeckung (10) auf dem Kopf (11) einer Person getragen wird, geeignet sind, die Ohren der Person zu bedecken, **dadurch gekennzeichnet, dass** jeder Ohrabschnitt (11) wenigstens ein Abdeckstück (12, 12' 12a, 12b) umfasst, das zwischen einem geöffneten Modus, in dem es einen Zugang einer Gehörschutzkapsel (16) durch eine Öffnung (14) in den Ohrabschnitten (11) der Kopfbedeckung auf eine solche Weite ermöglicht, dass Ränder der Gehörschutzkapseln fest an dem Kopf der dieselben tragenden Person anliegen, und einem geschlossenen Modus, in dem das Abdeckstück (12, 12', 12a, 12b) den Ohrabschnitt (11) bedeckt, um die Ohren der Person bedeckt zu halten, eingestellt werden kann.

2. Kopfbedeckung (10) nach Anspruch 1, wobei das Abdeckstück (12, 12', 12a, 12b) eine äußere Abfütterung, die so angeordnet ist, dass sie der Außenseite der Kopfbedeckung zugewandt ist, und eine innere Abfütterung, die so angeordnet ist, dass sie der Innenseite der Kopfbedeckung zugewandt ist, aufweist, und wobei verhindert wird, dass die innere Abfütterung sowohl in dem geschlossenen als auch dem geöffneten Modus der Au-ßenseite ausgesetzt ist.

3. Kopfbedeckung (10) nach Anspruch 3, wobei die innere Abfütterung des Abdeckstücks (12, 12' 12a, 12b) geeignet ist, dem Kopf des Trägers sowohl in dem geschlossenen als auch dem geöffneten Modus zugewandt zu sein.

4. Kopfbedeckung (10) nach einem der vorhergehenden Ansprüche, wobei wenigstens die Ohrabschnitte (11) der Kopfbedeckung (10) zwei Schichten umfassen, wobei eine Innenschicht (21) der zwei Schichten eine Öffnung (14) aufweist, die an die Größe einer Gehörschutzkapsel (16) angepasst ist, und wobei das Abdeckstück (12) eine Au-ßenschicht bildet, die die Öffnung (14) in dem geschlossenen Modus bedeckt, die aber in dem geöffneten Modus nach unten gezogen werden kann, um einen Zugang einer Gehörschutzkapsel (16) durch die Öffnung (14) in der Innenschicht (21) zu ermöglichen.

5. Kopfbedeckung (10) nach Anspruch 4, wobei die Au-ßenschicht eine zusätzliche Abfütterung umfasst, die so positioniert ist, dass sie eine zweite Schicht bereitstellt, die geeignet ist, die Öffnung (14) der Innenschicht (21) in dem geschlossenen Modus zu bedecken.

6. Kopfbedeckung (10) nach einem der Ansprüche 1, 2 oder 3, wobei jeder Ohrabschnitt (11) zwei Abdeckabschnitte (12a, 12b) umfasst, die in dem geschlossenen Modus aneinander gefügt werden können, um die Ohren (15) einer die Kopfbedeckung (10) tragenden Person zu bedecken, und die voneinander getrennt werden können, um einen Zugang einer Gehörschutzkapsel (16) zwischen den zwei Abdeckabschnitten (12a, 12b) zu ermöglichen.

7. Kopfbedeckung (10) nach Anspruch 6, wobei die Abdeckabschnitte (12a, 12b) Anbringungsstücke umfassen, um die Abdeckabschnitte (12a, 12b) aneinander anzubringen.

8. Kopfbedeckung (10) nach Anspruch 7, wobei die Anbringungsstücke Klettverschlüsse, Druckknöpfe, Hemdknöpfe und/oder Magnete umfassen.

9. Kopfbedeckung (10) nach Anspruch 6, wobei die Ohrabschnitte (11) einen Reißverschluss (19) umfassen, um die Abdeckabschnitte (12a, 12b) aneinander anzubringen, wobei der Reißverschluss (19) geöffnet werden kann, um einen Zugang einer Gehörschutzkapsel durch eine Reißverschlussöffnung zu ermöglichen, und geschlossen werden kann, um die Ohrabschnitte (11) zu bedecken und die Ohren einer die Kopfbedeckung tragenden Person abgedeckt zu halten.

10. Kopfbedeckung (10) nach Anspruch 9, wobei die Reißverschlussöffnung (14) einen Zungenteil (20) umfasst, der einen Endabschnitt der Reißverschlussöffnung (14) bedeckt.

11. Kopfbedeckung (10) nach einem der Ansprüche 9 oder 10, wobei der Reißverschluss (19) auf eine solche Weise bogenförmig ist, dass ein vorderer Abdeckabschnitt (12a) der zwei Abdeckabschnitte (12a, 12b) kleiner als ein hinterer Abdeckabschnitt (12b) ist.

12. Kopfbedeckung (10) nach einem der Ansprüche 1, 2 oder 3, wobei das Abdeckstück (12, 12') drehbar angeordnet ist und in einer geschlossenen Stellung, in der es eine Öffnung (14) des Ohrabschnitts (11) bedeckt, und einer geöffneten Stellung, in der es die Öffnung (14) nicht abdeckt, sondern einen Zugang einer Gehörschutzkapsel (16) durch die Öffnung (14) ermöglicht, positioniert werden kann.

13. Kopfbedeckung (10) nach Anspruch 12, wobei jedes Abdeckstück (12, 12') Anbringungsstücke (24a-f) umfasst, die ermöglichen, dass es an entsprechenden Anbringungsstücken (23a-f) befestigt werden kann, die an dem Ohrabschnitt (11) der Kopfbedeckung (10) angeordnet sind.

14. Kopfbedeckung (10) nach Anspruch 13, wobei jedes Abdeckstück (12, 12') sowohl in der geschlossenen als auch der geöffneten Stellung an den Anbringungsstücken (23a-f) des Ohrabschnitts (11) der Kopfbedeckung (10) befestigt werden kann.

15. Kopfbedeckung (10) nach einem der Ansprüche 12 bis 14, wobei jedes Abdeckstück (12, 12') um einen unteren Anbringungspunkt (23f, 24f) drehbar ist, der sich unter der Öffnung (14) des Ohrabschnitts (11) befindet.

## Revendications

1. Casque (10) conçu pour être porté avec et sans protège-oreilles (16), ledit casque (10) comprenant deux parties oreille opposées, qui, lorsque le casque (10) est porté sur la tête (11) d'une personne, sont conçues pour recouvrir les oreilles de ladite personne, **caractérisé en ce que** chaque partie oreille (11) comprend au moins une pièce de recouvrement (12, 12', 12a, 12b) qui peut être réglée entre un mode ouvert dans lequel elle permet l'accès à un protège-oreilles (16) à travers une ouverture (14) dans les parties oreille (11) du casque de sorte que les bords des protège-oreilles puissent reposer fermement contre la tête de la personne qui les porte, et un mode fermé dans lequel la pièce de recouvrement (12, 12', 12a, 12b) recouvre la partie oreille (11) de sorte à garder les oreilles de ladite personne recouvertes.

2. Casque (10) selon la revendication 1, la pièce de recouvrement (12, 12', 12a, 12b) ayant un revêtement extérieur conçu pour faire face à l'extérieur du casque et un revêtement intérieur conçu pour faire face à l'intérieur du casque, et le revêtement intérieur étant empêché d'être exposé à l'extérieur à la fois en mode fermé et en mode ouvert.

3. Casque (10) selon la revendication 3, le revêtement intérieur de la pièce de recouvrement (12, 12', 12a, 12b) étant conçu pour faire face à la tête de l'utilisateur aussi bien en mode fermé qu'en mode ouvert.

4. Casque (10) selon l'une quelconque des revendications précédentes, au moins les parties oreille (11) du casque (10) comprenant deux couches, une couche intérieure (21) des deux couches ayant une ouverture (14) adaptée à la taille d'un protège-oreilles (16), et la pièce de recouvrement (12) formant une couche extérieure qui recouvre ladite ouverture (14) dans le mode fermé mais qui peut être tirée vers le bas dans le mode ouvert de sorte à permettre l'accès à un protège-oreilles (16) à travers ladite ouverture (14) dans la couche intérieure (21).

5. Casque (10) selon la revendication 4, la couche extérieure comprenant un revêtement supplémentaire positionné de sorte à fournir une seconde couche conçue pour recouvrir l'ouverture (14) de la couche intérieure (21) dans le mode fermé.

6. Casque (10) selon l'une quelconque des revendications 1, 2 ou 3, chaque partie oreille (11) comprenant deux pièces de recouvrement (12a, 12b) qui peuvent être jointes l'une à l'autre dans ledit mode fermé afin de recouvrir les oreilles (15) d'une personne portant le casque (10) et qui peuvent être séparées l'une de l'autre de sorte à permettre l'accès à un protège-oreilles (16) entre lesdites deux pièces de recouvrement (12a, 12b).

7. Casque (10) selon la revendication 6, les pièces de recouvrement (12a, 12b) comprenant des pièces de fixation pour la fixation des pièces de recouvrement (12a, 12b) l'une à l'autre.

8. Casque (10) selon la revendication 7, les pièces de fixation comprenant une bande Velcro, des boutons-pressions, des boutons de chemise et/ou des aimants.

9. Casque (10) selon la revendication 6, les parties oreille (11) comprenant une fermeture à glissière (19) pour fixer les pièces de recouvrement (12a, 12b) l'une à l'autre, ladite fermeture à glissière (19) pouvant être ouverte pour permettre l'accès à un protège-oreilles par une ouverture de fermeture à glissière, et fermée pour recouvrir les parties oreille (11) de sorte à garder recouvertes les oreilles d'une personne portant le casque.

10. Casque (10) selon la revendication 9, l'ouverture à fermeture à glissière (14) comprenant une partie languette (20) qui recouvre une partie d'extrémité de l'ouverture à fermeture à glissière (14).

11. Casque (10) selon la revendication 9 ou 10, la fermeture à glissière (19) étant en forme d'arc, de sorte qu'une pièce de revêtement avant (12a) des deux pièces de recouvrement (12a, 12b) soit plus petite qu'une pièce de revêtement arrière (12b).

12. Casque (10) selon l'une quelconque des revendications 1, 2 ou 3, la pièce de recouvrement (12, 12') étant disposée de manière rotative et pouvant être positionnée dans une position fermée où elle recouvre une ouverture (14) de la partie oreille (11), et une position ouverte où elle ne recouvre pas l'ouverture (14) mais permet l'accès à un protège-oreilles (16) à travers ladite ouverture (14).

13. Casque (10) selon la revendication 12, chaque pièce de recouvrement (12, 12') comprenant des pièces de fixation (24a-f) qui permettent de la fixer à des pièces de fixation (23a-f) correspondantes disposées au niveau de la partie oreille (11) du casque (10).

14. Casque (10) selon la revendication 13, chaque pièce de couvercle (12, 12') pouvant être fixée à la fois dans la position fermée et dans la position ouverte aux pièces de fixation (23a-f) de la partie oreille (11) du casque (10).

15. Casque (10) selon l'une quelconque des revendications 12 à 14, chaque pièce de recouvrement (12, 12') pouvant tourner autour d'un point de fixation inférieur (23f, 24f) situé au-dessous de l'ouverture (14) de la partie oreille (11).
